# EUROPEAN PATENT SPECIFICATION

(11) **EP 4 351 702 B1**
(45) Date of publication and mention of the grant of the patent: **17.12.2025**
(21) Application number: 22732265.8
(22) Date of filing: 10.06.2022
(51) Int. Cl.: A61N 1/05, A61N 1/36, A61N 1/375, A61B 5/263, A61B 5/293

(54) **ELECTRODE DEVICE**
ELEKTRODENVORRICHTUNG
DISPOSITIF D'ÉLECTRODE

(30) Priority: 11.06.2021 EP 21382525
(43) Date of publication of application: 17.04.2024
(73) Proprietor: INBRAIN Neuroelectronics SL, 08028 Barcelona (ES)
(72) Inventor: DECRE, Michel, 5654 NC Eindhoven (NL); MAULL MIQUEL, Aina, 08041 Barcelona (ES); BAKKER, Bert, 4261 LH Wijk en Aalburg (NL)
(74) Representative: DTS Patent- und Rechtsanwälte PartmbB
(86) International application number: PCT/EP2022/065863
(87) International publication number: WO 2022/258818

(56) References cited:
- US-A1- 2011 230 747
- US-A1- 2018 243 550
- US-A1- 2021 138 230
- US-B1- 8 774 891

## Description

The present invention refers to an electrode device for a neurostimulation system, an electrode module, a neurostimulation system and a method for manufacturing the same.

The present invention relates to the technical field of neurostimulation, in particular to nerve stimulation and/or cortical stimulation and/or deep brain stimulation.

Particularly, cortical and/or deep brain Stimulation are methods for the diagnosis and therapy of neurodegenerative diseases such as *inter alia* the Parkinson's disease, epilepsy, and chronic pain. Electrical stimulation by means of leads which are implanted into brain areas or regions like the subthalamic nucleus and/or the globus pallidus internus can alleviate symptoms, such as tremor symptoms of a patient suffering from a drug-resistant Parkinson's disease. Further, the signals from a brain region or area at which the leads were implanted can be recorded and the state of the brain tissue can be determined using impedance measurements.

In this relation, neuroprosthetic devices are powerful tools to monitor, prevent and treat neural diseases, disorders and conditions by interfacing electrically with the nervous system. They are capable of recording and stimulating electrically neural activity once implanted in the nervous tissue. Currently, most neuroprosthetic technologies apply electrodes interfacing with neural tissue.

Classical electrode arrays based on metal electrodes or other high impedance and low charge injection capacity materials and require large surface electrodes to safely inject sufficient therapeutic current into the nervous tissue.

In more detail, standard commercially available neural interfaces are based on metallic microelectrodes made of platinum Pt, platinum-iridium (Pt/Ir), iridium oxide (IrOx) or titanium nitride (TiN). Those materials interact with the living tissue through a combination of Faradaic and capacitive currents, offer a limited chemical stability and are rigid. Metals' performance strongly drops in microelectrodes of tens of micrometres in diameter. Further, metals degrade under continuous tissue stimulation.

In order to avoid some of these disadvantages, electrodes with a coating applied to an electrode base material were suggested. However, such coatings bring along other disadvantages which include chemical and mechanical degradation of the coatings when the electrode is in operation.

In this regard graphene based (coating) materials are very promising. This is not only because these materials are highly inert and mechanically robust and therefore avoid degradation problems. Graphene based materials are highly favorable also because of their electrical/electrochemical properties and flexibility. Graphene based materials thus appear ideal for safe electrical interfacing in aqueous environments.

Moreover, neurostimulation leads, whether based on conventional ring- or segmented-metal electrode arrays or thin-film-based electrode arrays, also have the significant disadvantage that they are stiff at least by part, potentially limiting implantation options and increasing mechanical mismatch between the lead and nervous tissue, like e.g. brain tissue.

Examples for such leads are described in US 10,441,779 B2 and US 10,406,350 B2.

US2011/230747A1 discloses an implantable biomedical device comprising nanostructures single crystalline silicon or thin electrode arrays and a bioresorbable substrate, optionally in combination with a barrier layer having a mesh structure. The bioresorbable substrate, e.g., a silk substrate, provides support for the barrier layer during deployment, manipulation, positioning, and/or interfacing.

US2018/243550A1 discloses a device comprising a pattern of electrodes, e.g., round-dot-shaped or hexagonal-shaped electrodes, arranged on a base layer/substrate, separated by spaces.

It is an object of the invention to provide an electrode device which comprises improved performance with regard to the electrical properties, in particular of the electrode design, as well as providing a mechanically improved design of the electrode device in order to adapt it to the properties of human tissue by particularly enhancing flexibility and compatibility of the electrode device. Moreover, it is an object of the invention to provide a corresponding electrode module, a neurostimulation system as well as a method for manufacturing the same.

The aforementioned objects are solved by the subject-matters of the independent claims. Advantageous configurations of the invention are described in dependent claims.

According to the present invention an electrode device for a neurostimulation system, in particular for nerve stimulation and/or cortical stimulation and/or deep brain stimulation, is provided comprising a substrate and at least one electrode. The at least one electrode is arranged within the substrate and the substrate at least partially comprises a mesh-like structure.

The invention is based on the idea to apply flexibility, in particular internal flexibility, to the substrate of the electrode device by reducing the bulk material and thereby forming
a mesh-like structure, preferably a specifically designed mesh-like structure, in order to predetermine structural properties and behaviour of the electrode device under load scenarios.

If the mesh-like structures become too thin, the substrate loses structural stability and could become difficult to handle or assemble. However, by using openings/voids having the same/constant geometrical properties going from the millimetric (mm) to the micrometric (µm) range, the larger scaled remaining structures of the substrate can provide structural stability while smaller scaled remaining structures of the substrate allow for (local) flexibility and electrode routing.

However, by the openings/voids as provided further advantages can be obtained/provided/maintained, like a fluid pass through the structure and/or a tissue grow-in into the substrate having the mesh-like structure, thus allowing for an embedding of the substrate.

Moreover, the openings/voids may also allow for a needle or other instruments to be passed through the electrode device without affecting the electrode device, e.g. in the context of acute cortical stimulation/recording applications.

Preferably, the mesh-like structure is a specifically designed structure, comprising voids/holes with predetermined shape, size, amount and/or distribution, preferably to be evaluated along a cross-section of the electrode device. Thereby, the resulting mechanical properties as well as electrical properties concerning the at least one integrated electrode can be, at least to a certain extent, pre-set and pre-defined.

Moreover, the at least one electrode is preferably integrated inside the substrate's material. The voids/holes may surround the integrated electrode in a circumferential direction.

In particular in case of rod-like electrode, e.g. for deep brain stimulation, the voids can extend in a longitudinal direction of the electrode device and/or can be interrupted by substrate's material within certain intervals.

The electrode device can be specifically designed in its electrical and mechanical properties considering the respective application by appropriate configuration of the substrate's mesh-like structure, in particular concerning an adaption to the mechanical properties of brain tissue.

With regard to subacute, acute and/or chronic epicortical (epidural/subdural) stimulation and/or intracortical stimulation, in particular corresponding leads and other electrode devices like nerve cuffs, the amount of polymer film/substrate covering the brain can be reduced in order to maintain more healthy tissue as well as to enhance the conformability of the electrode array.

**In** consideration of electrode devices for deep brain stimulation the flexibility of the tip region, particularly interacting with the brain tissue, can be improved.

**In** one preferred embodiment of the invention, the at least one electrode comprises graphene, in particular is made of graphene, a graphene based material or comprises a coating made of graphene.

Such graphene electrodes particularly provide higher safe charge injection capacity as well as an improvement of the signal-to-noise ratio/performance. Thereby, the electrode size can be reduced, even if the same amount of electrodes is maintained. This is particularly favorable to the spatial resolution and localisation of signal detection or stimulation delivery. Moreover, small electrodes can also provide, for example and in contrast to large electrodes, access to high frequency applications.

Hence, along a cross-section of the electrode device the cross-sectional area of the at least one electrode can be reduced.

Moreover, such graphene electrodes also provide improved flexibility.

**In** particular, graphene offers superior performance even in thin material layers. Hence, the use of graphene (based) material may offer the possibility to use methods like photolithography or other patterned deposition techniques like screen printing, inkjet printing or 3D printing. **In** consequence, the manufacturing process of a substrate may be simplified.

**In** summary, the efficiency of the at least one electrode can be improved by also providing enhanced mechanical and structural properties not only of the electrode but of the overall electrode device.

According to a preferred embodiment of the invention, the mesh-like structure of the substrate comprises multiple voids, preferably of different sizes. In another embodiment of the invention, the voids comprise different size categories in the range of mm to µm, in particular at least 2, 4, 6 or 8 different size categories.

Preferably, the mesh-like structure is provided by forming a plurality of different voids along the substrate, in particular along a cross-section of the substrate. The mesh-like structure can be provided with voids having different size categories and being arranged in a specific pattern. Moreover, the specific pattern can belong to subgroups having symmetry orders of 2, 3, or 6 which are known to provide homogeneous tiling of surfaces, and provide flexibility in multiple directions.

In case of rod-like stimulation or recording electrodes devices such pattern can be identified and evaluated along respective cross-sections of the electrode device. Thereby, smaller and larger sized structure of the substrate can be formed. The void pattern can be provided, either extended or repeated, along/over a longitudinal extension of the rod-like electrode device.

Hence, the load distribution can be directed and controlled by the specific design of the substrate's mesh-like structure.

In another embodiment the voids are symmetrically distributed, in particular along a cross-section of the substrate, in order to provide isotropic properties of the electrode device.

For example, certain patterns formed of multiple different kinds of voids/holes can be replicated multiple times around a central longitudinal axis of the electrode device. Alternatively, the symmetry can be provided e.g. with respect to a certain symmetry plane or the like. Along a thin-film based electrode device, there can be multiple symmetry lines/planes in order to allow for multiple replications of the void pattern.

Preferably, the symmetry of the voids in the substrate is provided in a way such that isotropic mechanical properties can be achieved.

Such properties may be achievable by adapting the symmetry pattern to the outer shape of the electrode device, e.g. in case of a hexagonal outer shape of the electrode device by providing a void pattern which is replied six times around an axis of symmetry, wherein e.g. a three times replication may be applied in case of a triangular outer shape of the electrode device.

By achieving isotropic mechanical properties the electrode device provides beneficial handling during stimulation treatment, i.e. beneficial treatment options, and protects the nervous tissue due to its mechanical adaption.

According to one embodiment the voids of the substrate comprise a circular shape, a triangular shape, a hexagonal shape, an octagonal shape, a rectangular shape, a parallelogram shape, a squared shape and/or the like, in particular as a cross-sectional shape.

Preferably, the shape of the voids inside the substrate can be adapted to the respective setup/layout of the electrode device. In more detail, by different shapes of the voids, the load distribution inside and along the substrate, i.e. its remaining structures, can be designed in order to achieve a suitable behaviour, in particular isotropic or anisotropic mechanical properties, an adaptability to the properties of brain tissue etc.

In particular, the design of the voids can be aligned and coordinated with e.g. the outer shape of the electrode device and/or a specific setup/layout along a thin film structure of the electrode device.

The electrode device can be individually designed for allowing the suitable adaption to the respective application within neurostimulation.

In another preferred embodiment the electrode device comprises at least one stimulation electrodeand multiple recording electrodes.

The stimulation signals as induced and provided by the stimulation electrode of the electrode device can be recorded and monitored by the recording electrodes. Moreover, the recording electrodes can provide surveillance of the patient's health status and/or allow for monitoring and/or adaption of stimulation treatment.

Preferably the recording electrodes are arranged in a symmetrical pattern like the voids, in order to provide isotropic properties of the electrode device.

A beneficial surveillance, monitoring and treatment of the patient is available, e.g. due to the symmetrical data collection by the recording electrodes.

In one embodiment the electrode device comprises at least one conductive connector, preferably comprising gold, being situated along the electrode device, in particular along at least a part of the outer circumference of the electrode device.

By the conductive connector, an electrical signal along the outer circumference of the electrode device can be recorded, detected and forwarded along the conductive connector.

For example, the conductive connector may expand along a part of the circumference and/or in the longitudinal direction of a rod-like electrode device.

The conductive connector is provided as a connection means along the outer side of the electrode device, in particular within gaps being formed inbetween multiple electrode devices being arranged next to each other, in order to provide electrical contact to the stimulation and recording electrodes. In particular, the conductive connector can be arranged within the electrode module and inbetween the multiple electrode devices.

According to another embodiment, the electrode device comprises multiple conductive connection elements, preferably comprising gold, being positioned in and/or on the substrate for connecting each electrode with the conductive connector. In another preferred embodiment of the invention, each of the conductive connection elements are arranged along the voids of the substrate, preferably in a straight line between the voids, such that a connection angle between the conductive connection element and the conductive connector is the same for all conductive connection elements of the electrode device.

The conductive connection elements provide the electrical contact between the outer side of the electrode device, in particular to the conductive connector, and the electrodes within the substrate, in particular to the recording electrodes and/or to the stimulation electrode.

By ensuring that each conductive connection element comprises identical connection angle with respect to the corresponding part of the conductive connector, the same electrode flow/current behavior may be provided.

Moreover, the conductive connector can represent a combination of the single conductive connection elements. The conductive connection elements may be continued as separate connection lines/traces within the conductive connector respectively. In this context, the connection angle can be considered as the angle at which the respective conductive connection elements enters/aligns with the conductive connector.

Alternatively, the conductive connector can represent a common conductive line/trace to which each of the conductive connection elements of the respective electrode device is connected.

The electrode device according to the invention can preferably provide isotropic behaviour with respect to its mechanical properties and its electrical properties.

In another embodiment, the electrode device comprises a circular outer shape, a triangular outer shape, a hexagonal outer shape, an octagonal outer shape, a rectangular outer shape, a parallelogram outer shape, a squared outer shape and/or the like, in particular a respective cross-section, or a (thin) film structure.

Also the outer shape of the electrode device can be adapted to the specific circumstances of the stimulation treatment in order to achieve the best possible adaption of the electrical and mechanical performance of the device.

The outer shape of the electrode device may be selected and coordinated with respect to the architecture of the substrate, namely the mesh-like structure of the substrate, or vice versa.

The electrode device according to the invention represents a highly adjustable/adaptable and pre-definable/pre-configurable device for neurostimulation in order to define its electrical and mechanical properties, preferably isotropic properties, to and in accordance with the specific field of application.

According to another aspect of the invention, an electrode module for a neurostimulation system is provided, comprising at least one electrode device according to the present invention.

Preferably the electrode module is provided with a plurality of electrode devices. Multiple electrode devices can be used in the form of a single electrode device, thus in synchronized manner, and/or as single devices respectively being coordinated with each other.

In one preferred embodiment of the invention, multiple electrode devices are arranged side by side, wherein the multiple electrode devices each comprise a conductive connector, wherein the conductive connectors are combined in order to form a conductive connection expanding along and/or between the different electrode devices, in particular at least a part of the outer circumferences of the electrode devices.

Hence, the multiple electrode devices can function and be applied as a common unit, wherein electrical signals being detected via the conductive connectors, which are combined with each other, are forwarded to all of the electrode devices being grouped together in terms of an electrode module.

In particular, multiple conductive connectors can be combined together and continued as separate conductive connectors in such combined arrangement in order to provide a conductive connection.

Alternatively, the multiple conductive connectors can be interconnected and formed together as a single line/trace as conductive connection.

The arrangement of the conductive connectors may provide more mechanical balance and the possibility to fine tune the fractal design at the border of each electrode device to protect the conductive connectors and/or the conductive connection elements from mechanical stress.

A beneficial use of a plurality of electrode devices, compiled together as an electrode module, can be provided and applied for neurostimulation, e.g. a specifically coordinated use of the multiple electrode devices and/or specific mechanical properties being achieved by a combination of differently designed electrode devices, in particular electrode devices with differently designed substrates having mesh-like structures.

According to another embodiment, the electrode module comprises at least two electrode devices, wherein the second electrode device is arranged, preferably concentrically arranged, at a longitudinal end of the first electrode device such that a multi-layer/multi-stack arrangement of electrode devices is provided. In a further preferred embodiment the at least one electrode of the second electrode device is concentrically arranged with a void of the mesh-like structure of the first electrode device.

The first and second electrode devices can be provided in a stacked arrangement.

In particular, the electrode module can provide multi-layer/multi-stack stimulation and/or recording, a higher resolution, additional steering options, an embedding in the (nervous) tissue.

According to another embodiment the electrode module comprises at least one sensor device, preferably an optical sensor device, being arranged at and/or along a void of the at least one electrode device such that the nervous tissue can be observed via the void by the sensor device.

In particular, the sensor device may be capable of observing/monitoring the nervous tissue through a void of the mesh-like structure of the at least one electrode device.

For example, the sensor device may be configured to observe/determine a colour of the nervous tissue, preferably to asses the viability of the tissue. Hereby, also the positioning of the electrode module may be supported and enhanced.

According to another aspect of the invention a neurostimulation system is provided, in particular for nerve stimulation and/or cortical stimulation and/or deep brain stimulation, comprising at least one electrode device or at least one electrode module according to the present invention.

A further aspect of the invention refers to a method for manufacturing an electrode device and/or an electrode module and/or a neurostimulation system.

In particular, the process to provide voids/holes inside the substrate can be individually adapted in order to pre-define the electrical and/or mechanical properties of the single electrode device in consideration of the respective application.

Further details and advantages of the present invention shall now be disclosed in the connection with the drawings.

### It is shown in:

- **Fig. 1**: perspective view of a schematic embodiment of an electrode module;
- **Fig. 2**: detailed cross-section of an embodiment according to **Fig. 1****;** and
- **Fig. 3**: perspective schematic illustration of a second embodiment of an electrode module.

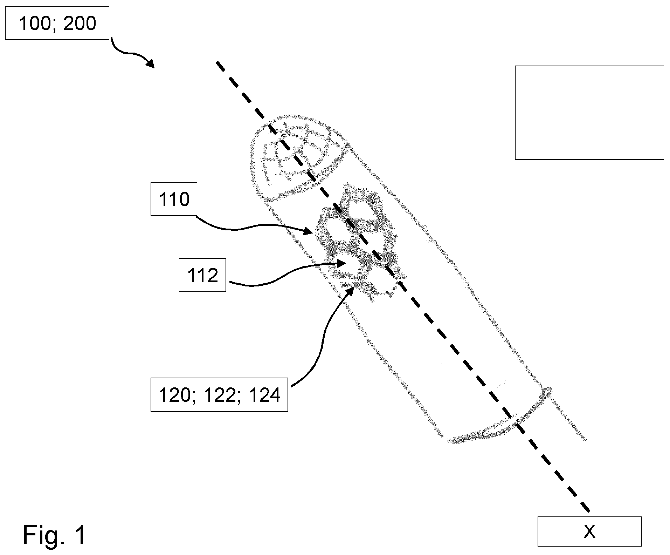

**Fig. 1** illustrate a first schematic embodiment of an electrode module 200 with at least one electrode device 100.

According to **Fig. 1** the electrode module 200, and the corresponding electrode device 100, is provided in a rod-like shape comprising a longitudinal axis X.

In a tip region of the electrode device 100 a substrate 110 is provided.

Inside the substrate 110 multiple electrodes 120 are arranged.

At least one stimulation electrode 122 is provided. Moreover, a plurality of recording electrodes 124 is arranged within substrate.

The electrode 120; 122; 124 can be a graphene electrode, in particular comprising graphene (based) material and/or comprising a graphene (based) coating.

The substrate comprises a plurality of voids 112.

In particular, the voids 112 form a certain pattern, due to its size, distribution and shape. The voids 112 cause the substrate to form a mesh-like structure.

According to the embodiment in **Fig. 1****,** the mesh-like structure of the substrate 110 ends at the tip of the rod-like electrode device 100, such that the tip may be provided in form of a (classical) stimulation electrode 122, i.e. with substrate bulk material or the like.

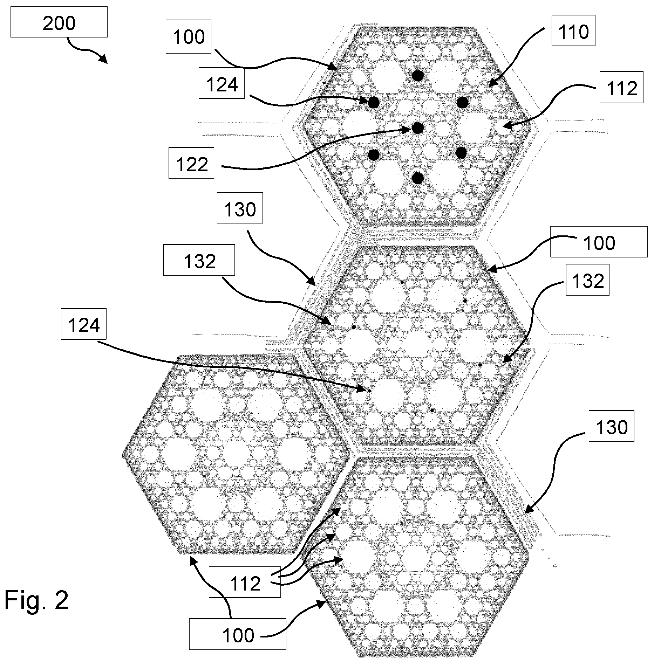

**Fig. 2** illustrates a detailed cross-section of an embodiment according to **Fig. 1****.**

In particular, the electrode devices 100 are provided in **Fig. 2** in different configurations for illustrative purpose.

The electrode devices 100 comprise a hexagonal outer shape.

Alternatively, the electrode device 100 can be provided in a shape of a triangular, a square, an octagon, a parallelogram or the like.

Multiple electrode devices 100 are arranged next to each other along one of their flat outer sides respectively.

The electrode device 100 particularly comprises a substrate 110 and at least one electrode 120, preferably one stimulation electrode 122 and multiple recording electrodes 124.

The stimulation electrode 122 may arranged in the middle of the electrode device 100, along the longitudinal axis of the electrode device's 100 rod-like shape.

According to **Fig. 2****,** six recording electrodes 124 are provided within the substrate 110, symmetrically surrounding and evenly distributed around the stimulation electrode 122.

The lowest electrode device 100 in **Fig. 2** particularly illustrates the configuration of the substrate 110.

The voids 112 of the substrate 110 comprise a hexagonal shape, preferably in conformity with the outer shape of the electrode device 100.

The substrate comprises a symmetric arrangement of the voids 112.

According to **Fig. 2** the symmetry is provided with respect to the longitudinal axis of the respective electrode device 100.

The voids 112 are provided in different sizes, namely four different size categories.

In particular, differently sized voids 112 are mixed together and form a group of voids 112.

Preferably, the groups of differently sized voids 112 are evenly distributed around the longitudinal axis of the electrode device 100.

In respect of the hexagonal outer shape of the electrode device 100, the group of voids 112 is replicated six times around the symmetry/longitudinal axis.

The size of the voids 112 decreases from a more central region of the substrate 110 to an outer region of the substrate 110.

Alternatively, the differently sized voids 112 can be distributed differently, e.g. increase from a more central region of the substrate 110 to an outer region of the substrate 110.

As a further alternative, the differently sized voids may also be distributed in a non-symmetrical manner in order to modify/enhance the electrode device's 100 properties, e.g. being/compression properties, in a specific direction and/or at a specific side of electrode device 100 and the substrate 110.

In **Fig. 2** the electrode devices 100 of the electrode module 200 can comprise conductive connectors 130.

The conductive connector 130 of each electrode device 100 can extend around a part of the circumference of the respective electrode device 100.

In particular, the conductive connectors 130 can extend between two electrode devices 100 being situated next to each other.

The conductive connectors 130 are provided/arranged inside of the electrode module 200.

The conductive connectors 130 of the various electrode devices 100 can becombined/assembled with each other in order to be continued as separate conductive connectors in assembled form.

Alternatively, the conductive connectors 130 may be interconnected with each other, thus (functionally) forming a common conductive connector 130.

On the inside of the substrate, conductive connection elements 132 can extend from the outer circumference of the respective electrode device 100 to the electrodes 120; 122; 124.

In particular, the recording electrodes 124 can be connected to the conductive connector 130 via the conductive connection elements 132.

The stimulation electrode 122 can be connected to the conductive connector 130 via a separate conductive connection element 132.

In general, conductive connection elements 132 can extend along and/or between the voids 112 of the substrate 110.

Preferably, the conductive connection elements 132 may be embodied as straight lines extending along and/or between the voids 112 of the substrate 110 from the outer circumference of the electrode device 100 to the respective electrode 120; 122; 124.

The conductive connection elements 132 can comprise one or multiple bend portions, for interconnecting straight line parts in order to allow for appropriate extension through the substrate 110.

The conductive connection elements 132 of one electrode device 100 comprise a constant/identical connection angle with respect to the respective part of the conductive connector 130.

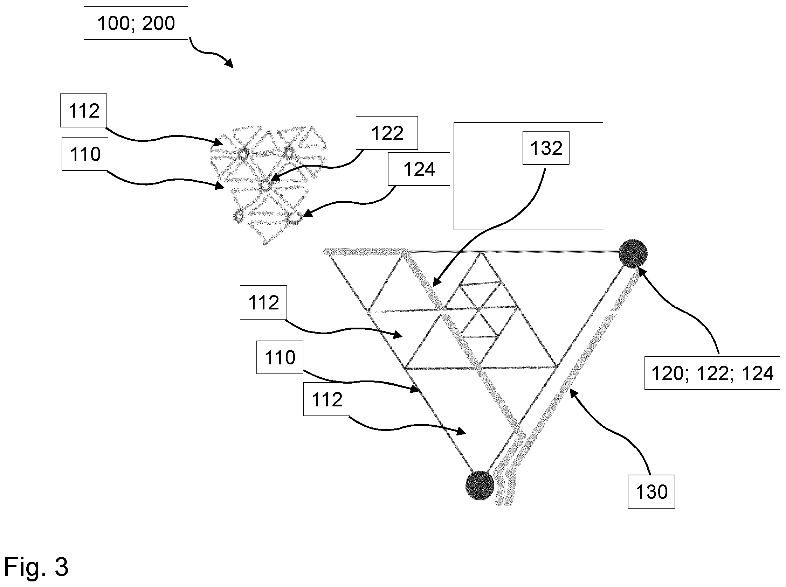

In **Fig. 3** a perspective schematic illustrations of a second embodiment of an electrode module 200 with at least one electrode device 100 are shown.

**Fig. 3** illustrates an embodiment of an electrode device 100 based on/comprising a (thin) film structure, e.g. for cortical stimulation treatment and/or for a nerve cuff or the like.

The (thin) film structure comprises a substrate 110.

The substrate 110 is provided with a plurality of voids 112.

The voids 112 may have a triangular shape.

The triangular shaped voids 112 are arranged such that the substrate 110 may form a contiguous, symmetric structure.

Within the substrate 110, multiple electrodes 120 are provided, in particular at least one stimulation electrode 122 and multiple recording electrodes 124.

Preferably, the electrodes 120 are arranged in a way that a certain pattern is provided.

For example, the stimulation electrode 122 can be arranged in the middle, while at least four recording electrodes 124 are arranged around/in proximity of the stimulation electrode 122 at a respective distance of one void 112.

Alternatively, six recording electrodes may be arranged around the stimulation electrode 122 at a respective distance of one void 112.

Along the remaining structures of the substrate 110, being formed by the voids 112, conductive connectors 130 may be provided.

Conductive connection elements 132 can be provided as extension of the conduction connection 130 in order to electrically connect all of the electrodes 120, at least the recording electrodes 124, appropriately.

The function of an electrode device 100 as illustrated in Fig. 1 to 3 can be described as follows.

With particular respect to the embodiment pursuant to Fig. 1 and 2, the stimulation electrode 122 and the multiple recording electrodes 124 are integrated in the substrate 110.

The symmetric and grouped arrangement of voids/holes 112, comprising different size categories, provide isotropic mechanical properties of the electrode device 100 and the electrode module 200.

The mesh-like structure of the substrate 110 does cause a higher flexibility of the electrode device 100 and can enhance the usage during treatment.

Thicker, larger remaining structures of the substrate 110 can provide structure, mechanical stability.

Thinner, smaller remaining structures of the substrate 110 can provide flexibility of the electrode device 100.

In particular, the voids 112 may be configured and arranged such that the electrode device 100 comprises isotropic mechanical properties.

Due to the isotropic properties, the use of the electrode device 100 and the corresponding electrode module 200 is independent from its orientation.

According to Fig. 3, the voids 112 may be arranged in a non-symmetrical manner.

By a non-symmetrical design of the electrode device 100, orientation-specific properties, e.g. bending/compression properties or the like, can be provided.

By the conductive connection elements 132 comprising constant/the same connection angles with respect to the respective conductive connector 130, all electrodes 120; 122; 124 comprise the same electrode flow/current properties.

Preferably the mesh-like structure of the substrate 110 and the conductive connection elements 132 cause mechanical and electrical properties which may provide an isotropic behaviour of the electrode device 100 and the corresponding electrode module 200.

Alternatively, the mesh-like structure of the substrate 110 can be adapted in order to provide specific anisotropic behaviour.

In summary, the present invention provides an electrode device 100 which comprises improved mechanical properties, in particular enhanced flexibility, in order to support the neurostimulation treatment procedure by adapting to the human nerve tissue, in particular brain tissue. Preferably, the electrode device can provide isotropic mechanical properties to facilitate the treatment independent from an orientation of the electrode device 100.

Moreover, also electrical properties of the electrode device 100 can be enhanced, e.g. by appropriate orientation of electrical conductive connection elements 132 in order to improve and unify electrode flow/current behaviour.

Such electrical properties can particularly be further improved by the implementation of graphene electrodes, which allow for smaller electrode sizes, higher safe charge injection capacity and an improved signal-to-noise ratio.

### Reference numerals

- 100: Electrode device
- 110: substrate
- 112: void
- 120: electrode
- 122: stimulation electrode
- 124: reading electrode
- 130: conductive connector
- 132: conductive connection element
- 200: Electrode module
- X: Longitudinal axis

## Claims

1. Electrode device (100) for a neurostimulation system, in particular for nerve stimulation and/or cortical stimulation and/or deep brain stimulation, comprising a substrate (110) and at least one electrode (120),
wherein the at least one electrode (120) is arranged within the substrate (110),
wherein the substrate (110) at least partially comprises a mesh-like structure, the mesh-like structure of the substrate (110) comprising multiple voids (112), **characterized in that** the voids (112) of the mesh-like structure comprise a triangular shape, a hexagonal shape, or an octagonal shape, in particular as cross-sectional shape.

2. Electrode device (100) according to claim 1,
**characterized in that**
the at least one electrode (120; 122; 124) comprises graphene, in particular is made of graphene, a graphene-based material or comprises a coating made of graphene.

3. Electrode device (100) according to one of the preceding claims,
**characterized in that**
the multiple voids (112) of the mesh-like structure have different sizes.

4. Electrode device (100) according to claim 3,
**characterized in that**
the voids (112) comprise different size categories in the range of mm to µm, in particular at least 2, 4 or 6 different size categories.

5. Electrode device (100) according to one of the preceding claims,
**characterized in that**
the voids (112) are symmetrically distributed, in particular along a cross-section of the substrate (110), in order to provide isotropic properties of the electrode device (100).

6. Electrode device (100) according to one of the preceding claims,
**characterized in that**
the electrode device (100) comprises at least one stimulation electrode (122) and multiple recording electrodes (124).

7. Electrode device (100) according to one of the preceding claims,
**characterized in that**
the electrode device (100) comprises at least one conductive connector (130), preferably comprising gold, being situated along the electrode device (100), in particular along at least a part of the outer circumference of the electrode device (100).

8. Electrode device (100) according to one of the preceding claims,
**characterized in that**
the electrode device (100) comprises multiple conductive connection elements (132), preferably comprising gold, being positioned in and/or on the substrate (110) for connecting each electrode (120; 122; 124) with the conductive connector.

9. Electrode device (100) according to one of the preceding claims,
**characterized in that**
each of the conductive connection elements (132) are arranged along the voids (112) of the substrate (110), preferably in a straight line between the voids (112), such that a connection angle between the conductive connection element (132) and the conductive connector (130) is the same for all conductive connection elements (132) of the electrode device (100).

10. Electrode device (100) according to one of the preceding claims,
**characterized in that**
the electrode device (100) comprises a circular outer shape, a triangular outer shape, a hexagonal outer shape, an octagonal outer shape or the like, in particular a respective cross-section, or a film structure.

11. Electrode module (200) for a neurostimulation system comprising at least one electrode device (100) according to one of the preceding claims.

12. Electrode module (200) according to claim 11,
**characterized in that**
multiple electrode devices (100) are arranged side by side,
wherein the multiple electrode devices (100) each comprises a conductive connector (130), wherein the conductive connectors (130) are combined in order to form a conductive connection expanding along and/or between the different electrode devices (100), in particular at least a part of the outer circumferences of the electrode devices (100).

13. Electrode module (200) according to claim 11 or 12,
**characterized in that**
the electrode module (200) comprises at least two electrode devices (100), wherein the second electrode device (100) is arranged, preferably concentrically arranged, at a longitudinal end of the first electrode device (100) such that a multi-layer arrangement of electrode devices (100) is provided.

14. Electrode module (200) according to claim 13,
**characterized in that**
the at least one electrode (120) of the second electrode device (100) is concentrically arranged with a void (112) of the mesh-like structure of the first electrode device (100).

15. Electrode module (200) according to one of claims 11 to 14,
**characterized in that**
the electrode module (200) comprises at least one sensor device, preferably an optical sensor device, being arranged at and/or along a void (112) of the at least one electrode device (100) such that the nervous tissue can be observed via the void (112) by the sensor device.

16. Neurostimulation system, in particular for nerve stimulation and/or cortical stimulation and/or deep brain stimulation, comprising at least one electrode device (100) or at least one electrode module (200) according to one of the preceding claims.

17. Method for manufacturing an electrode device (100) and/or an electrode module (200) and/or a neurostimulation system according to one of the preceding claims.

## Patentansprüche

1. Elektrodenvorrichtung (100) für ein Neurostimulationssystem, insbesondere zur Nervenstimulation und/oder kortikalen Stimulation und/oder tiefen Hirnstimulation, einen Träger (110) und mindestens eine Elektrode (120) aufweisend,
wobei die mindestens eine Elektrode (120) innerhalb des Trägers (110) angeordnet ist und der Träger (110) zumindest teilweise eine gitterartige Struktur aufweist, wobei die gitterartige Struktur des Trägers (110) zahlreiche Lücken (112) aufweist, **dadurch gekennzeichnet, dass**
die Lücken (112) der gitterartigen Struktur eine dreieckige Form, eine sechseckige Form oder eine achteckige Form, insbesondere als Querschnittsform, aufweisen.

2. Elektrodenvorrichtung (100) nach Anspruch 1,
**dadurch gekennzeichnet, dass**
die mindestens eine Elektrode (120; 122; 124) Graphen aufweist, insbesondere aus Graphen oder einem auf Graphen basierenden Material hergestellt ist, oder eine Beschichtung aus Graphen aufweist.

3. Elektrodenvorrichtung (100) nach einem der vorhergehenden Ansprüche, **dadurch gekennzeichnet, dass**
die zahlreichen Lücken (112) der gitterartigen Struktur unterschiedliche Größen haben.

4. Elektrodenvorrichtung (100) nach Anspruch 3,
**dadurch gekennzeichnet, dass**
die Lücken (112) unterschiedliche Größenkategorien im Bereich von mm bis µm aufweisen, insbesondere mindestens 2, 4 oder 6 unterschiedliche Größenkategorien.

5. Elektrodenvorrichtung (100) nach einem der vorhergehenden Ansprüche, **dadurch gekennzeichnet, dass**
die Lücken (112) symmetrisch verteilt sind, insbesondere entlang eines Querschnitts des Trägers (110), um isotrope Eigenschaften der Elektrodenvorrichtung (100) bereitzustellen.

6. Elektrodenvorrichtung (100) nach einem der vorhergehenden Ansprüche, **dadurch gekennzeichnet, dass**
die Elektrodenvorrichtung (100) mindestens eine Stimulationselektrode (122) und zahlreiche Aufzeichnungselektroden (124) aufweist.

7. Elektrodenvorrichtung (100) nach einem der vorhergehenden Ansprüche, **dadurch gekennzeichnet, dass**
die Elektrodenvorrichtung (100) mindestens einen leitfähigen Verbinder (130) aufweist, der vorzugsweise Gold aufweist und sich entlang der Elektrodenvorrichtung (100) befindet, insbesondere entlang mindestens eines Teils des Außenumfangs der Elektrodenvorrichtung (100).

8. Elektrodenvorrichtung (100) nach einem der vorhergehenden Ansprüche, **dadurch gekennzeichnet, dass**
die Elektrodenvorrichtung (100) zahlreiche leitfähige Verbindungselemente (132) aufweist, die vorzugsweise Gold aufweisen und in und/oder auf dem Träger (110) zur Verbindung jeder Elektrode (120; 122; 124) mit dem leitfähigen Verbinder positioniert sind.

9. Elektrodenvorrichtung (100) nach einem der vorhergehenden Ansprüche, **dadurch gekennzeichnet, dass**
die leitfähigen Verbindungselemente (132) jeweils entlang den Lücken (112) des Trägers (110) angeordnet sind, vorzugsweise in einer geraden Linie zwischen den Lücken (112), und zwar derart, dass ein Verbindungswinkel zwischen dem leitfähigen Verbindungselement (132) und dem leitfähigen Verbinder (130) für alle leitfähigen Verbindungselemente (132) der Elektrodenvorrichtung (100) identisch ist.

10. Elektrodenvorrichtung (100) nach einem der vorhergehenden Ansprüche, **dadurch gekennzeichnet, dass**
die Elektrodenvorrichtung (100) eine kreisförmige Außenform, eine dreieckige Außenform, eine sechseckige Außenform, eine achteckige Außenform oder dergleichen, insbesondere einen jeweiligen Querschnitt, oder eine Filmstruktur aufweist.

11. Elektrodenmodul (200) für ein Neurostimulationssystem, mindestens eine Elektrodenvorrichtung (100) nach einem der vorhergehenden Ansprüche aufweisend.

12. Elektrodenmodul (200) nach Anspruch 11,
**dadurch gekennzeichnet, dass**
zahlreiche Elektrodenvorrichtungen (100) nebeneinander angeordnet sind,
wobei die zahlreichen Elektrodenvorrichtungen (100) jeweils einen leitfähigen Verbinder (130) aufweisen, wobei die leitfähigen Verbinder (130) verknüpft sind, um eine leitfähige Verbindung zu bilden, die sich entlang und/oder zwischen den unterschiedlichen Elektrodenvorrichtungen (100), insbesondere mindestens über einen Teil der Außenumfänge der Elektrodenvorrichtungen (100), ausbreitet.

13. Elektrodenmodul (200) nach Anspruch 11 oder 12,
**dadurch gekennzeichnet, dass**
das Elektrodenmodul (200) mindestens zwei Elektrodenvorrichtungen (100) aufweist, wobei die zweite Elektrodenvorrichtung (100) vorzugsweise konzentrisch an einem Längsende der ersten Elektrodenvorrichtung (100) derart angeordnet ist, dass eine mehrschichtige Anordnung aus Elektrodenvorrichtungen (100) bereitgestellt ist.

14. Elektrodenmodul (200) nach Anspruch 13,
**dadurch gekennzeichnet, dass**
die mindestens eine Elektrode (120) der zweiten Elektrodenvorrichtung (100) zu einer Lücke (112) der gitterartigen Struktur der ersten Elektrodenvorrichtung (100) konzentrisch angeordnet ist.

15. Elektrodenmodul (200) nach einem der Ansprüche 11 bis 14,
**dadurch gekennzeichnet, dass**
das Elektrodenmodul (200) mindestens eine Sensorvorrichtung aufweist, vorzugsweise eine optische Sensorvorrichtung, die an und/oder entlang einer Lücke (112) der mindestens einen Elektrodenvorrichtung (100) derart angeordnet ist, dass über die Lücke (112) durch die Sensorvorrichtung das Nervengewebe beobachtet werden kann.

16. Neurostimulationssystem, insbesondere zur Nervenstimulation und/oder kortikalen Stimulation und/oder tiefen Hirnstimulation, das mindestens eine Elektrodenvorrichtung (100) oder mindestens ein Elektrodenmodul (200) nach einem der vorhergehenden Ansprüche aufweist.

17. Verfahren zur Herstellung einer Elektrodenvorrichtung (100) und/oder eines Elektrodenmoduls (200) und/oder eines Neurostimulationssystems nach einem der vorhergehenden Ansprüche.

## Revendications

1. Dispositif d'électrodes (100) pour un système de neurostimulation, en particulier pour la stimulation nerveuse et/ou la stimulation corticale et/ou la stimulation cérébrale profonde, comprenant un support (110) et au moins une électrode (120),
ladite au moins une électrode (120) étant disposée à l'intérieur du support (110) et le support (110) présentant au moins en partie une structure en forme de grille, la structure en forme de grille du support (110) présentant de nombreux interstices (112), **caractérisé en ce que**
les interstices (112) de la structure en forme de grille présentent une forme triangulaire, une forme hexagonale ou une forme octogonale, en particulier en section transversale.

2. Le dispositif d'électrodes (100) selon la revendication 1,
**caractérisé en ce que**
ladite au moins une électrode (120 ; 122 ; 124) comprend du graphène, est notamment fabriquée à partir de graphène ou d'un matériau à base de graphène, ou comprend un revêtement en graphène.

3. Le dispositif d'électrodes (100) selon l'une quelconque des revendications précédentes,
**caractérisé en ce que**
les nombreux interstices (112) de la structure en forme de grille ont des tailles différentes.

4. Le dispositif d'électrodes (100) selon la revendication 3,
**caractérisé en ce que**
les interstices (112) ont différentes catégories de tailles dans la plage de mm à µm, en particulier au moins 2, 4 ou 6 catégories de tailles différentes.

5. Le dispositif d'électrodes (100) selon l'une quelconque des revendications précédentes,
**caractérisé en ce que**
les interstices (112) sont répartis symétriquement, en particulier le long d'une section transversale du support (110), afin de conférer des propriétés isotropes au dispositif d'électrodes (100).

6. Le dispositif d'électrodes (100) selon l'une quelconque des revendications précédentes,
**caractérisé en ce que**
le dispositif d'électrodes (100) comprend au moins une électrode de stimulation (122) et de nombreuses électrodes d'enregistrement (124).

7. Le dispositif d'électrodes (100) selon l'une quelconque des revendications précédentes,
**caractérisé en ce que**
le dispositif d'électrodes (100) comporte au moins un connecteur conducteur (130) comprenant de préférence de l'or et étant situé le long du dispositif d'électrodes (100), en particulier le long d'au moins une partie de la périphérie extérieure du dispositif d'électrodes (100).

8. Le dispositif d'électrodes (100) selon l'une quelconque des revendications précédentes,
**caractérisé en ce que**
le dispositif d'électrodes (100) comprend de nombreux éléments de connexion conducteurs (132) comprenant de préférence de l'or et étant positionnés dans et/ou sur le support (110) pour connecter chaque électrode (120 ; 122 ; 124) avec le connecteur conducteur.

9. Le dispositif d'électrodes (100) selon l'une quelconque des revendications précédentes,
**caractérisé en ce que**
les éléments de connexion conducteurs (132) sont disposés respectivement le long des interstices (112) du support (110), de préférence en ligne droite entre les interstices (112), de telle sorte qu'un angle de connexion entre l'élément de connexion conducteur (132) et le connecteur conducteur (130) soit identique pour tous les éléments de connexion conducteurs (132) du dispositif d'électrodes (100).

10. Le dispositif d'électrodes (100) selon l'une quelconque des revendications précédentes,
**caractérisé en ce que**
le dispositif d'électrodes (100) présente une forme extérieure circulaire, une forme extérieure triangulaire, une forme extérieure hexagonale, une forme extérieure octogonale ou similaire, en particulier une section transversale respective, ou une structure en film.

11. Module d'électrodes (200) pour un système de neurostimulation, comportant au moins un dispositif d'électrodes (100) selon l'une quelconque des revendications précédentes.

12. Le module d'électrodes (200) selon la revendication 11,
**caractérisé en ce que**
de nombreux dispositifs d'électrodes (100) sont disposés les uns à côté des autres, les nombreux dispositifs d'électrodes (100) comportant chacun un connecteur conducteur (130), les connecteurs conducteurs (130) étant reliés pour former une connexion conductrice qui s'étend le long et/ou entre les différents dispositifs d'électrodes (100), en particulier au moins sur une partie des périphéries extérieures des dispositifs d'électrodes (100).

13. Le module d'électrodes (200) selon la revendication 11 ou 12,
**caractérisé en ce que**
le module d'électrodes (200) comprend au moins deux dispositifs d'électrodes (100), le deuxième dispositif d'électrodes (100) étant de préférence disposé de manière concentrique par rapport à une extrémité longitudinale du premier dispositif d'électrodes (100) de telle sorte qu'un agencement multicouche de dispositifs d'électrodes (100) soit fourni.

14. Le module d'électrodes (200) selon la revendication 13,
**caractérisé en ce que**
ladite au moins une électrode (120) du deuxième dispositif d'électrodes (100) est disposée de manière concentrique par rapport à un interstice (112) de la structure en forme de grille du premier dispositif d'électrodes (100).

15. Le module d'électrodes (200) selon l'une quelconque des revendications 11 à 14,
**caractérisé en ce que**
le module d'électrodes (200) comporte au moins un dispositif capteur, de préférence un dispositif capteur optique, qui est disposé au niveau et/ou le long d'un interstice (112) dudit au moins un dispositif d'électrodes (100) de telle sorte que le tissu nerveux puisse être observé à travers l'interstice (112) par le dispositif capteur.

16. Système de neurostimulation, en particulier pour la stimulation nerveuse et/ou la stimulation corticale et/ou la stimulation cérébrale profonde, comprenant au moins un dispositif d'électrodes (100) ou au moins un module d'électrodes (200) selon l'une quelconque des revendications précédentes.

17. Procédé de fabrication d'un dispositif d'électrodes (100) et/ou d'un module d'électrodes (200) et/ou d'un système de neurostimulation selon l'une quelconque des revendications précédentes.
